(19) Europäisches Patentamt
European Patent Office
Office européen des brevets

(11) **EP 1 976 573 B1**

(12) **FASCICULE DE BREVET EUROPEEN**

(45) Date de publication et mention de la délivrance du brevet:
**29.08.2012 Bulletin 2012/35**

(21) Numéro de dépôt: **06847144.0**

(22) Date de dépôt: **26.12.2006**

(51) Int Cl.:
*A61L 15/46* (2006.01)       *A01N 59/16* (2006.01)
*A61L 15/18* (2006.01)       *A01N 55/02* (2006.01)
*A61L 26/00* (2006.01)

(86) Numéro de dépôt international:
**PCT/FR2006/002880**

(87) Numéro de publication internationale:
**WO 2007/074241 (05.07.2007 Gazette 2007/27)**

(54) **COMPLEXE D'ARGENT ET SON UTILISATION POUR PRODUIT D'HYGIENE OU DE SANTE**

SILBERKOMPLEX UND SEINE VERWENDUNG FÜR EIN GESUNDHEITS- ODER HYGIENEPRODUKT

SILVER COMPLEX AND USE THEREOF FOR A HEALTH OR HYGIENE PRODUCT

(84) Etats contractants désignés:
**DE ES GB IT**

(30) Priorité: **28.12.2005 FR 0513418**

(43) Date de publication de la demande:
**08.10.2008 Bulletin 2008/41**

(73) Titulaire: **Les Laboratoires Brothier**
**92000 Nanterre (FR)**

(72) Inventeurs:
• **MAINGAULT, Philippe**
**49700 Doue la Fontaine (FR)**
• **SEROUSSI, Cyril**
**49160 Saint Martin de la Place (FR)**

• **BRUNEL, Florence**
**49400 Saumur (FR)**

(74) Mandataire: **Bloch, Gérard et al**
**Gevers France**
**23bis, rue de Turin**
**75008 Paris (FR)**

(56) Documents cités:
**GB-A- 2 408 206**

• **Y. FUJITA, I. MORI, T. YAMAGUCHI:**
**"Spectrophotometric Determination of Biologically Active Thiols with Eosin, Silver(I) and Adenine" ANALYTICAL SCIENCES, vol. 18, 2002, pages 981-985, XP002393667 cité dans la demande**

**Description**

L'argent (Ag) possède des propriétés antibactériennes très intéressantes dans le cadre du traitement des plaies infectées. Cependant, son principal inconvénient est la très faible stabilité de sa forme active, à savoir l'argent ionique, notamment vis-à-vis de la lumière.

L'invention de la présente demande concerne la stabilisation de l'argent par complexation et l'utilisation du complexe résultant dans les articles d'hygiène et de santé, et notamment les pansements.

Plusieurs technologies d'intégration de l'argent dans les pansements sont connues.

1) <u>Revêtement des matériaux</u> : dépôt superficiel d'argent sur un substrat par la mise en oeuvre de techniques très diverses comme le plasma ou la sorption. Le document US6767551 enseigne un tel procédé. Toutefois, l'argent n'est libéré dans son intégralité que dans un délai inférieur à quelques heures, ce qui est trop bref et donc incompatible avec une utilisation de type pansement ou même implant, applications nécessitant le maintien de l'activité bactériostatique ou bactéricide entre 12 heures et plusieurs jours. Par ailleurs, l'argent étant libéré de manière très rapide et intense, il peut entraîner la présence d'un pic de concentration, engendrant ainsi un effet cytotoxique.

2) <u>Extrusion de mélanges</u> de sels polysaccharidiques d'argent partiels ou totaux comme par exemple un alginate mixte d'argent et de calcium. Le document WO2004024197 enseigne un tel procédé. Toutefois, le produit de la réaction est un complexe d'argent dont l'inconvénient réside dans sa très faible stabilité, même dans le cas des sels très partiels. Dans une variante, on peut procéder par incorporation, dans la matrice du pansement, de complexes ou actifs à base d'argent comme par exemple le zirconium d'argent, comme enseigné par le document GB2383047, ou la sulfadiazine argentique. Bien que cette méthode permette de s'affranchir de la matrice, son inconvénient principal est directement lié à la nature du composé dépendant de l'argent utilisé et peut donc parfois s'avérer très complexe en terme de technologie d'incorporation et de coloration finale.

3) <u>Inclusion d'argent métallique</u> dans les produits de pansement. Cette technologie met en oeuvre des agents de dispersion de particules micrométriques ou nanométriques d'argent, comme enseigné par le document WO05073289, mais malheureusement, dans ce cas, l'argent n'est pas disponible sous forme active et présente donc une activité faible, voire nulle, vis-à-vis des microorganismes.

4) Le document GB 248206 décrit un pansement anti-bactérien comprenant de l'argent ionique stabilisé.

Il faut par ailleurs intégrer l'inconvénient principal de l'argent : sa couleur, variant du gris-anthracite au noir, peu stable.

Le problème principal à résoudre lorsqu'on souhaite faire un pansement à base d'argent est donc, d'une part, de libérer de l'argent ionique dans l'environnement du pansement et, d'autre part, de stabiliser l'argent et sa couleur.

Qu'il s'agisse de la technique de revêtement, de l'extrusion d'un complexe particulier ou de l'inclusion d'argent métallique, l'argent est intégré au pansement sous une forme non satisfaisante, pour continuer avec l'exemple du pansement.

L'invention de la présente demande vise donc à proposer un complexe d'argent satisfaisant.

Ainsi, l'invention de la présente demande concerne un produit d'hygiène ou de santé anti-bactérien comprenant un substrat et un complexe d'argent comprenant

- de l'argent ionique,
- un composé A choisi dans l'ensemble des purines et des composés thiolés,
- un composé B choisi dans l'ensemble des xanthènes,
- le composé A et l'argent ionique étant, dans le complexe, dans un rapport molaire de 1/2 à 2/1.

Le complexe de l'invention est donc un complexe ternaire de coordination qui présente les avantages suivants :

- argent stabilisé et notamment photostabilisé ;
- argent disponible pour son interaction avec les bactéries dans le microenvironnement du substrat auquel le complexe ternaire est intégré ;
- maintien de l'efficacité de la forme ionique de l'argent ;
- complexe apportant une fonction colorante.

De préférence, le composé xanthénique est l'éosine Y, mais il peut aussi s'agir de la fluorescéïne.

L'éosine B et un mélange de dérivés xanthéniques sont aussi envisageables.

De préférence, le composé A est choisi dans l'ensemble comprenant l'adénine (vitamine B 14), la guanine, le glutathion, la cystéine.

L'adénine conduit à un complexe très stable.

De préférence, l'argent est sous forme d'un sel, de préférence encore le nitrate.

On remarquera que l'invention de la présente demande concerne un complexe et non une association de composés, un complexe étant défini par une structure moléculaire et une seule, ce qui n'est pas le cas d'une association.

Il existe des documents enseignant l'association de deux des composés du complexe de l'invention de la présente demande (argent, éosine et adénine), mais tous relatifs à des applications qui n'ont rien à voir avec l'hygiène et la santé. Ainsi, EP 1 500 978 concerne les semi-conducteurs, US 4 484 925, le traitement des cuirs.

Il existe aussi un document qui concerne le complexe de l'invention, à savoir l'article de FUJITA Y., MORI I. and YAMAGUCHI T.; Spectrophotometric détermination of biologically active thiols with eosin, silver (I) and adenine, Analytical Science ; 2002, vol 18. Mais ce document n'est pas une antériorité, car il concerne tout autre chose. L'objectif poursuivi dans cet article est l'utilisation du complexe ternaire formé *in situ* et extemporanément dans la détection et le dosage de thiols. Au contraire, le complexe de l'invention a pour objectif, s'agissant d'une application à un produit d'hygiène ou de santé, non pas d'attirer les thiols du substrat sur l'argent du complexe mais, à l'inverse, de provoquer un transfert de l'argent sur les thiols des bactéries pour bloquer le mécanisme de transport membranaire des bactéries et les tuer.

C'est tout le mérite de la demanderesse d'avoir proposé cette fonction inverse.

De préférence, le produit de l'invention est un pansement, avantageusement avec un substrat d'alginate.

Lors de l'utilisation du pansement, l'argent ne se libère dans le milieu qu'en présence de liquide, en l'occurrence biologique, et de contamination bactérienne par interaction avec les fonctions thiols des bactéries, produisant ainsi un effet statique ou biocide.

Avantageusement, et pour, dans le cas d'un pansement fibrillaire d'alginate, que la concentration de l'argent ionique dans le liquide biologique soit de 350 à 500 ppm, la concentration massique du complexe ternaire dans le substrat est de 1 à 5 %.

La concentration massique du complexe ternaire dans le substrat peut varier de manière importante, en fonction de la nature de ce dernier et de sa forme galénique, dans une plage de 1 % pour des crèmes, à 80 % pour des formes lyophilisées.

$$O$$
$$O\ O$$

La formation du complexe ternaire de coordination de l'invention s'effectue en deux temps. La première étape se déroule en solution et consiste à mettre en présence l'argent ionique, de préférence sous la forme d'un nitrate, avec le composé A. L'interaction, plus ou moins forte selon la base purique employée, se traduit par la formation d'un précipité colloïdal volumineux dont la couleur dépend du composé A employé. Cette interaction n'est que partielle et le rendement de la réaction ne peut donc jamais atteindre 100 %.

La stoechiométrie final de l'interaction argent ionique / composé A est bien sûr fonction des conditions opératoires, mais il est plus intéressant dans le cas des produits de pansement de privilégier l'ajout majoritaire d'argent. Ensuite, le composé B est ajouté soit sous forme solide, soit en solution dans le milieu réactionnel précédent. De nouvelles interactions s'établissent entre ce dérivé et l'argent ionique plus ou moins associé, se traduisant par la formation d'un précipité colloïdal suspendu en solution dont la couleur résultante est issue majoritairement de la composante B du complexe (molécule au pouvoir colorant). L'argent se trouve donc lié par l'intermédiaire d'interactions de coordination et se trouve ainsi stabilisé au sein d'une structure peu sensible à la lumière.

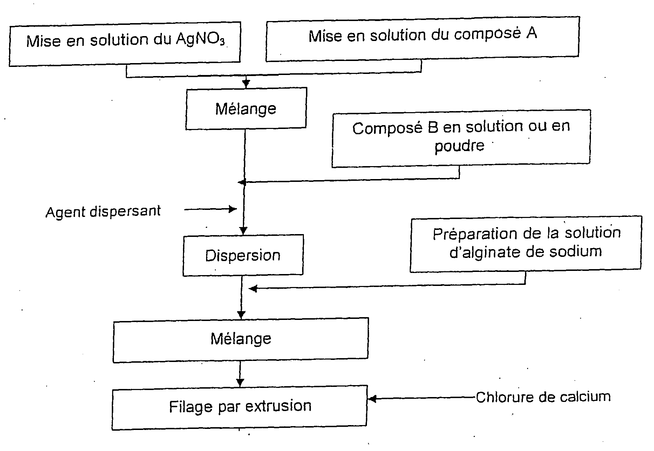

Dans le cas d'un pansement à base d'un alginate de calcium, le complexe ternaire est incorporé en suspension à une solution d'alginate de sodium. Le mélange obtenu est extrudé dans un bain calcique de manière à former la matrice d'alginate de calcium incorporant l'argent stabilisé dans le complexe ternaire. Le process peut être schématiquement illustré par l'organigramme de la figure unique en annexe et décrit comme suit.

Le nitrate d'argent et le composé A sont mis en solution dans de l'eau séparément, puis les deux solutions sont mélangées avant d'y introduire le composé B, en solution ou en poudre, dans le mélange. On procède à la dispersion du mélange par un agent dispersant, par exemple le polyvinylpyrolidone (PVP). On mélange ensuite le mélange précédent et une solution d'alginate de sodium pour obtenir un mélange final. On soumet ce mélange final à un filage par extrusion dans un bain de chlorure de calcium. On obtient ainsi un pansement à coloration stable et agréable.

L'utilisation du complexe de coordination stabilisant l'argent n'est pas réservée aux produits de pansement et peut ainsi être généralisée aux produits d'hygiène et de santé. Ainsi, le complexe en suspension peut être intégré à diverses formules :

- complexe ternaire formulé dans les produits cosmétiques quelle que soit la forme considérée (formes solides, liquides et pâteuses). Dans ce cas, l'intérêt est double puisque le complexe ternaire apporte des fonctions à la fois vis-à-vis de la charge microbienne et à la couleur finale de la composition, élément important, notamment dans les produits de maquillage, comme par exemple une crème contre l'acné ;

- complexe ternaire formulé dans les soins des plaies cutanées : au-delà de son intégration dans les pansements, l'argent stabilisé peut être utilisé avec avantage dans des compositions pâteuses ou liquides de traitement des plaies cutanées comme, par exemple, les crèmes cicatrisantes ou les films occultant les plaies (pansements liquides) ;

- plus généralement, complexe entrant dans la composition des dispositifs médicaux implantables ou non. Qu'ils soient stériles ou non, ces produits sont le plus souvent utilisés sur des « terrains » septiques ou apportant par leur mode d'insertion une biocharge. Ainsi, l'ajout de l'argent stabilisé dans la matrice des dispositifs médicaux pourrait éviter bon nombre de conséquences, parfois dramatiques, générées par la présence d'une flore pathogène. Exemples : argent stabilisé dans le matériau constitutif des cathéters, argent stabilisé et incorporé dans des colles tissulaires, ...

- utilisation du complexe d'argent stabilisé dans les articles d'hygiène comme les semelles antibactériennes, articles textiles (ex : chaussettes), lingettes anti-bactériennes.

## Revendications

1. Produit d'hygiène et de santé anti-bactérien comportant un substrat et un complexe d'argent comprenant

 - de l'argent ionique,
 - un composé A choisi dans l'ensemble des purines et des composés thiolés,
 - un composé B choisi dans l'ensemble des xanthènes,
 - le composé A et l'argent ionique étant, dans le complexe, dans un rapport molaire de 1/2 à 2/1.

2. Produit selon la revendication 1, dans lequel le composé xanthénique est choisi dans l'ensemble comprenant l'éosine B, l'éosine Y, le fluorescéïne, les dérivés xanthéniques et les mélanges de ces composés.

3. Produit selon l'une des revendications 1 et 2, dans lequel le composé A est choisi dans l'ensemble comprenant l'adénine, la guanine, le glutathion, la cystéine.

4. Produit selon l'une des revendications 1 à 3, dans lequel l'argent est sous forme de sel.

5. Produit selon la revendication 4, dans lequel il est prévu du nitrate d'argent.

6. Produit selon l'une des revendications 1 à 5, qui est un pansement.

7. Produit selon la revendication 6, comprenant un substrat d'alginate.

## Claims

1. Anti-bacterial hygiene and health product comprising a substrate and a silver complex containing

 - ionic silver,
 - a compound A selected from the group consisting of purines and thiol compounds,

 - a compound B selected from the group consisting of xanthens,
 - the compound A and the ionic silver being in a molar ratio of 1:2 to 2:1 in the complex.

2. Product according to claim 1, wherein the xanthenic compound is selected from the group consisting of eosin B, eosin Y, fluorescein, xanthenic derivatives and the mixtures of these compounds.

3. Product according to either claim 1 or claim 2, wherein the compound A is selected from the group consisting of adenine, guanine, glutathione, cysteine.

4. Product according to any of claims 1 to 3, wherein the silver is in the form of a salt.

5. Product according to claim 4, wherein silver nitrate is provided.

6. Product according to any of claims 1 to 5, which is a dressing.

7. Product according to claim 6, containing an alginate substrate.

## Patentansprüche

1. Antibakterielles Hygiene- und Gesundheitsprodukt, aufweisend ein Substrat und einen Silberkomplex, enthaltend:

 - ionisches Silber,
 - eine Verbindung A, die aus der Gruppe der Purine und der Thiolverbindungen gewählt ist,
 - eine Verbindung B, die aus der Gruppe der Xanthene gewählt ist,
 - wobei die Verbindung A und das ionische Silber in dem Komplex in einem Molverhältnis von 1/2 bis 2/1 vorhanden sind.

2. Produkt nach Anspruch 1, wobei die Xanthenverbindung aus der Gruppe enthaltend Eosin B, Eosin Y, Fluorescein, Xanthen-Derivate und Mischungen dieser Verbindungen gewählt ist.

3. Produkt nach einem der Ansprüche 1 und 2, wobei die Verbindung A aus der Gruppe enthaltend Adenin, Guanin, Glutathion und Cystein gewählt ist.

4. Produkt nach einem der Ansprüche 1 bis 3, wobei das Silber in Form von Salzen vorliegt.

5. Produkt nach Anspruch 4, wobei Silbernitrat vorgesehen ist.

6. Produkt nach einem der Ansprüche 1 bis 5, das ein Verband ist.

7. Produkt nach Anspruch 6, das ein Alginatsubstrat enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6767551 B
- WO 2004024197 A
- GB 2383047 A
- WO 05073289 A
- GB 248206 A
- EP 1500978 A
- US 4484925 A

**Littérature non-brevet citée dans la description**

- **FUJITA Y. ; MORI I. ; YAMAGUCHI T.** Spectrophotometric détermination of biologically active thiols with eosin, silver (I) and adenine. *Analytical Science,* 2002, vol. 18